Europäisches Patentamt

(19) European Patent Office

Office européen des brevets

(11) Publication number: **0 251 806 B1**

## EUROPEAN PATENT SPECIFICATION

(45) Date of publication of patent specification: **22.09.93**    (51) Int. Cl.⁵: **A61K 37/02**, //(A61K37/02, 31:725)

(21) Application number: **87305893.7**

(22) Date of filing: **03.07.87**

(54) **Plasminogen activator production.**

(30) Priority: **03.07.86 US 882060**

(43) Date of publication of application:
**07.01.88 Bulletin 88/01**

(45) Publication of the grant of the patent:
**22.09.93 Bulletin 93/38**

(84) Designated Contracting States:
**CH DE FR GB IT LI NL**

(56) References cited:
**EP-A- 0 142 344**
**EP-A- 0 186 084**
**EP-A- 0 219 270**

**ADVANCES IN BIOTECHNOLOGICAL PRO-CESSES, vol. 5, 1985, pages 275-299, Alan R. Liss, Inc.; A. KADOURI et al.: "Production of plasminogen activator by cells in culture"**

**BIOCHEMISTRY, vol. 24, no. 19, 10th September 1985, pages 4969-4973, American Chemical Society; R.R. LOBB et al.: "Induction of angiogenesis by bovine brain derived class 1 heparin-binding growth factor"**

**JOURNAL OF CELL BIOLOGY, vol. 99, no. 4,**

**part 2, October 1984, Abstracts of the American Society for Cell Biology, 24th Annual Meeting, Kansas City, Missouri, 12th - 16th November 1984, page 274a, Rockefeller University Press; S.M. DANILOV et al.: "Stimulation of cultured human vascular endothelial cell proliferation by growth factors from human brain heparin and thrombin"**

(73) Proprietor: **MERCK & CO. INC.**
**126, East Lincoln Avenue P.O. Box 2000**
**Rahway New Jersey 07065-0900(US)**

(72) Inventor: **Thomas, Kenneth A.**
**245 Washington Avenue**
**Chatham New Jersey 07928(US)**

(74) Representative: **Cole, William Gwyn et al**
**European Patent Department, Merck & Co., Inc., Terlings Park, Eastwick Road**
**Harlow, Essex CM20 2OR (GB)**

**Description**

Thrombosis, a formation of a clot within a blood vessel, gives rise to thrombotic strokes, deep vein thrombosis, myocardial infarction and other medical conditions which gives rise to necrosis of tissues and often times death of a patient. Even if death does not occur, thrombotic attacks are accompanied by damage to cells to which circulation has been prevented by thrombi formation. For recovery, it is necessary to dissolve the thrombi and further to repair the exposed cell surface after removal of thrombi by replacing the damaged non-thrombogenic vascular endothelial cells that form a monolayer lining healthy blood vessels in order to inhibit further clot formation. Thereafter, new tissue growth may be promoted by cellular proliferation.

If a thrombotic event has not yet occurred but is expected, clot formation may be prevented by the administration of anticoagulants. Heparin has been used to prevent clot formation. However, heparin has not been successfully employed to induce production of clot dissolving enzymes.

Fibroblast growth factor (FGF) activity has been found in bovine brain and pituitary gland, and mitogenic properties thereof have been recognized for a number of years, Thomas et al., Trends in Biochem. Sci. 11, 81 (1986). By 1980, an active FGF with an apparent acidic isoelectric point had been identified, Thomas, et al., J. Biol. Chem. 225, 5517 (1980). Subsequently, a highly purified acidic FGF (aFGF) was reported, U.S. Patent 4,444,760, April 24, 1984; Thomas, et al., Proc. Natl. Acad. Sci., USA 81, 357 (1984). More recently, there has been reported the complete amino acid sequence of aFGF from bovine brain, Giminez-Gallego et al., Science 220, 1385 (1985), the purification, characterization and amino terminal sequence from human brain Giminez-Gallego et al., Biochem. Biophys. Res. Commun. 135, 541 (1986). Although the mitogenic activity of aFGF is known, the property of inducing the expression of a plasminogen activator to have the net effect of digesting blood clots, is not known.

EP-A-0219270, which was published on 22nd April 1987, i.e. subsequent to the priority date of the present application, describes a method of enhancing the in vitro production of tissue plasminogen activator (t-PA) and single chain urokinase plasminogen activator (SCU-PA) by normal human diploid lung fibroblast cells in a serum-free medium, involving the addition of both heparin and an endothelial cell growth factor (such as aFGF) to the culture medium.

According to the present invention it has been discovered that acidic fibroblast growth factor (aFGF) causes degradation of fibrin in tissue culture, a property adaptable as a means for treating patients suffering from thrombotic attacks. When aFGF is employed together with heparin a significant increase in activity is noted. Thus, in a preferred embodiment of this invention, the administration is carried out together with heparin.

The present invention is based on the discovery that acidic fibroblast growth factor induces in tissue culture the expression of plasminogen activator, a proteolytic enzyme that converts the enzymatically inactive circulating plasma protein plasminogen to active plasmin, a protease which digests blood clots. This property may be adapted for decreasing the incidence and/or severity of subsequent clot formation in patients recovering from thrombosis. Moreover, the administration of aFGF decreases the incidence of subsequent clot formation and promotes healing by replacing damaged cells inasmuch as clot formation does not occur on the non-thrombogenic surface of healthy vascular endothelial cells. In addition, healing is further promoted by the known mitogenic activity of aFGF which then would promote growth of new cells. Thus, the invention comprises a method for inducing the production of vascular endothelial cell plasminogen activator thereby decreasing the incidence and/or the severity of subsequent clot formation in patients recovering from thrombosis and for promoting healing of the damaged endothelium by replacing damaged vascular endothelial cells by proliferation resulting from administering a therapeutically effective composition comprising aFGF. It has further been discovered that when aFGF is employed together with heparin, the plasminogen activator activity is significantly increased at the lower concentrations of the growth factor. This is unexpected since heparin alone, does not demonstrate this property to any significant extent. Thus, administration with heparin constitutes a preferred embodiment of this invention.

The aFGF useful in the process of the present invention is a protein which may be obtained from brain and other organs of bovine, human and other species, and when obtained from bovine or human brain has been characterized by the amino acid sequences hereinafter detailed. The bovine brain-derived aFGF may be prepared as described in U.S. Patent 4,444,760. The complete amino acid sequence has been determined and is reported in a publication Thomas et al., Science, 230, 1385-1388 (1985). The human aFGF may be obtained employing the process described by Conn, et al. Biochem. Biophys. Res. Commun. 124, 262-268 (1984), followed by purification employing $C_4$ HPLC (high pressure liquid chromatography) and analyzed for purity by electrophoresis in SDS (sodium dodecyl sulfate) polyacrylamide gels followed by silver staining as described in the article by Thomas et al., Proc. Natl. Acad. Sci. USA 81, 357-361 (1984) and Giminez-Gallego et al., Biochem. Biophys. Res. Commun. 135, 541-548 (1986). The amino acid composition was determined by hydrolyzing the purified protein forming derivatives with phenylisothiocyanate, chromatographing the resulting derivatives on a $C_{18}$ Zorbax column (DuPont) using a commercial (Varian 5500) liquid chromatograph to pump a gradient and quantitating using a commercial (Nelson analytical 4400) recording integrator.

The amino acid sequence of bovine aFGF was determined from the amino terminal sequence and overlapping peptides generated by cleavages with trypsin, Staphylococcus aureus V8 Protease, hydroxylamine, and cyanogen bromide. The carboxyl terminal sequence of the whole protein was confirmed by timed carboxypeptidase A digestion as reported in the Science 230, 1385 (1985) article.

The amino acid sequence of human aFGF was determined similarly from the amino terminal sequence and overlapping peptides generated by cleavages with trypsin, Staphylococcus aureus V8 protease and cyanogen bromide. The carboxyl terminal sequence of the whole protein was confirmed by carboxypeptidase A digestion (Giminez-Gallego et al., submitted for publication). Both the human and bovine aFGF amino acid sequences are shown below. The human aFGF (HaFGF) sequence is written in full. Only the differences in the bovine (BaFGF) sequence are listed below the corresponding residues of the human sequence.

```
                           1                          10
         HaFGF: PHE-ASN-LEU-PRO-PRO-GLY-ASN-TYR-LYS-LYS-PRO-LYS-LEU-LEU-TRY-

         BaFGF:                   -LEU-

                           20                                        30
                CYS-SER-ASN-GLY-GLY-HIS-PHE-LEU-ARG-ILE-LEU-PRO-ASP-GLY-THR-

                           -TYR-

                                             40
                VAL-ASP-GLY-THR-ARG-ASP-ARG-SER-ASP-GLN-HIS-ILE-GLN-LEU-GLN-

                            -LYS-

                           50                                        60
                LEU-SER-ALA-GLU-SER-VAL-GLY-GLU-VAL-TYR-ILE-LYS-SER-THR-GLU-

                    -CYS-             -ILE-

                                             70
                THR-GLY-GLN-TYR-LEU-ALA-MET-ASP-THR-ASP-GLY-LEU-LEU-TYR-GLY-

                            -PHE-

                           80                                        90
                SER-GLN-THR-PRO-ASN-GLU-GLU-CYS-LEU-PHE-LEU-GLU-ARG-LEU-GLU-

                                            100
                GLU-ASN-HIS-TYR-ASN-THR-TYR-ILE-SER-LYS-LYS-HIS-ALA-GLU-LYS

                           110                                      120
                ASN-TRP-PHE-VAL-GLY-LEU-LYS-LYS-ASN-GLY-SER-CYS-LYS-ARG-GLY-

                HIS-                              -ARG-SER-   -LEU-

                                            130
                PRO-ARG-THR-HIS-TYR-GLY-GLN-LYS-ALA-ILE-LEU-PHE-LEU-PRO-LEU-

                            -PHE-

                                            140
                PRO-VAL-SER-SER-ASP
```

The 140-residue bovine and human aFGF sequences are 92 percent identical (only 11 amino acid substitutions).

Heparin is a sulfated glycosaminoglycan consisting of equal parts of the sugars D-glucosamine and D-glucuronic acid which are sulfated to varying degrees. It is commercially available in unmodified form as well as in a solution form for direct therapeutic utilization.

The usefulness of aFGF in digesting thrombi may be demonstrated by the ability of aFGF to bring about fibrinolysis. The property may be observed in a test in which aFGF induces expression of plasminogen activators by endothelial cells thereby causing lysis of [$^{125}$I]fibrin with release of radioactive fragments which can be recovered from the assay medium and determined for extent of radioactivity. For such a determination, endothelial cells can be prepared from bovine adrenal cortex following the method of Folkman et al., Proc. Natl. Acad. Sci. USA 76, 5217 (1979). In such a procedure, finely cut pieces of adrenal cortex obtained under sterile conditions from adrenal glands of calves are incubated in 0.5 percent collagenase to release capillary endothelial cells and capillary segments. The cells then may be grown in alpha minimal essential medium containing 5 percent calf serum and antibiotics, cloned and grown to confluence. Thereafter, the cells are treated with aFGF in the presence or absence of heparin, rinsed with phosphate-buffered saline and solubilized with detergent buffered to a pH of about 8. The solubilized cell extract modified by adding serum albumin and plasminogen is assayed for plasminogen activator activity using the [$^{125}$I]fibrin digestion.

The [$^{125}$I]fibrin digestion method of J.C. Unkeless et al., J. Expt. Med. 137, 85 (1973) is used to determine capacity of an agent to cause fibrinolysis. Plasminogen activators are proteolytic enzymes that convert the inactive plasma proenzyme plasminogen to the active protease plasmin. A coupled enzyme assay is used to measure proteolytic degradation by plasmin of [$^{125}$I]fibrin to produce soluble radiolabeled fragments of fibrin. In such an assay method, bacteriological plates coated with [$^{125}$I]fibrinogen in phosphate buffered saline are dried and thereafter incubated in nutrient medium supplemented with serum whereupon fibrinogen is converted into insoluble fibrin, leaving plates covered with [$^{125}$I]fibrin. The solubilized cell extract then may be plated onto the culture dishes, allowed to incubate for a period of 1 to 2 hours; an aliquot of assay mixture taken and soluble plasmin digested [$^{125}$I]fibrin fragments quantitated using a gamma spectrometer. The results of such tests may be seen in the working examples subsequently described.

The composition of the present invention utilizes the aFGF to induce vascular repair and expression of plasminogen activator for digesting clots and inhibiting subsequent clot formation in thrombotic patients by intravenously administering a therapeutic dose of the aFGF, as the drug, or a composition comprising same. In general, the dose may be that to provide between 1 microgram/kilogram/day to 100 milligrams/kilogram/day while considering patient's health, weight, age, and other factors which influence drug response. The drug may be administered either by a single injection, multiple injections or continuous infusion.

In the composition of the present invention, aFGF is present with heparin. When aFGF is to be administered with heparin, the same dose of aFGF may be employed as without heparin and the dose of heparin may be that generally administered to prevent thrombotic attack. It is most desirable to provide aFGF in the dose range of 10μg - 10mg/kg/day and heparin in the range of 70 - 1400 U.S.P. units/kg/day.

Compositions to be employed in the practice of the present invention comprises acidic fibroblast growth factor and heparin in sterile physiological media. A therapeutic composition may contain from 1 μg to 100 mg of acidic growth factor and 70 to 1400 units of heparin. Since 140 units of heparin is about 1 mg, the composition may contain from 1 μg to 100 mg of aFGF and 0.5 to 10 mg heparin of composition. Such compositions may also contain other ingredients for purposes such as for aiding solubility or for preservation acceptable for intravenous administration. The compositions may be prepared on a larger scale or as concentrate compositions which may be appropriately diluted for use.

The foregoing has been described in terms of therapeutic application to human patients, the method is also useful for similar application to all mammals. Thus, the process of the present invention may also be utilized in veterinary medicine for animal patients.

The following examples illustrate the invention.

EXAMPLE I

Preparation of Capillary Endothelial Cells for Assay.

Capillary endothelial cells were prepared by a modification of the procedure of Folkman et al. Proc. Natl. Acad. Sci. 76, 5217 (1979). Adrenal cortex obtained from adrenal glands of calves was cut into 1

4

millimeter cubic pieces, rinsed in phosphate-buffered saline (0.01 M phosphate buffer, pH 7.4, 0.15 M sodium chloride) and centrifuged. The pellet comprising the cortical segments was then incubated in 0.5 percent bacterial collagenase at room temperature for about 1 hour followed by repeated pipeting to disperse the tissue in smaller pieces. The suspension was filtered through 110 $\mu$m nylon mesh (Nitex, Elmford, NY) and the filtrate centrifuged at 650 rpm for 7 minutes at 4°C to obtain a pellet containing principally capillary segments and endothelial cells. The pellet was resuspended in Dulbecco's modified Eagle medium* supplemented with calf serum to 10 percent by volume and washed 3 times by centrifugation and resuspension in fresh volumes in this medium. The cells were finally centrifuged and the pellet then was resuspended in modified Eagle's medium, plated onto gelatin-coated dishes and incubated t 37°C. The dishes were previously coated from a 1 percent (weight/volume) gelatin (Difco, Detroit, MI) solution in calcium and magnesium-free phosphate-buffered saline,** allowed to stand at 4°C overnight, the gelatin aspirated and dishes washed once with modified Eagle medium immediately prior to plating the cells. Capillary segments and aggregates of endothelial cells were found to adhere to the substratum while adrenal cortical cells and fibroblasts remained in the supernatant and were removed by aspirating the supernatant between 1 and 3 hours after plating. Contamininating non-endothelial cells were manually removed and individual endothelial cell colonies expanded and cloned. The cells were grown in minimum essential medium alpha*** containing 5 percent calf serum and 100 units of penicillin and 100 micrograms of streptomycin per milliliter. Cells were passaged by trypsinization and subcultured at 20 percent their confluent density.

Cloned bovine capillary vascular endothelial cells (passages 5-10) were plated on 35 millimeter diameter tissue culture dishes and grown to confluence (about 750,000 cells per dish). The cells were then rinsed 3 times and scraped off the dish in cold phosphate-buffered saline, centrifuged at 400 x g for 10 minutes and the pellets solubilized in 0.25 percent Triton® X-100 detergent buffered with 0.1 M Tris-HCl (tris(hydroxymethyl)amiomethane, pH adjusted with HCl) to pH 8.1. The solubilized cells were used to assay for plasminogen activator activity.

Plasminogen Activator Activity Assay

[$^{125}$I]fibrinogen was prepared from commercial crude bovine fibrinogen (Miles, Naperville, IL). The crude fibrinogen was further purified by precipitation according to the method of Laki, Arch. Biochem. Biophys. 32, 317 (1951) and iodinated according to the procedure of Helmkamp et al., Cancer Res. 20, 1945 (1960). A three-fold molar excess of ICl relative to fibrinogen was added to 2-10 mCi of carrier-free [$^{125}$I]Na and this was added rapidly to about 10 milligrams of fibrinogen dissolved in 1 milliliter of phosphate-buffered saline. The product was passed over a column of Dowex-I-Cl$^-$ to remove free iodine, dialyzed against 2X calcium and magnesium-free phosphate-buffered saline to obtain [$^{125}$I]fibrinogen and frozen in 1 milliliter aliquots.

[$^{125}$I]fibrin-coated non-sterile 96 well (16 mm diameter wells) plastic bacteriological plates were prepared by coating each well with 30 micrograms of fibrinogen containing 40,000 cpm of [$^{125}$I]fibrinogen in 250 microliters of 0.1X phosphate-buffered saline and dried at 37°C for 24 hours. [$^{125}$I]fibrin was generated from fibrinogen on the surfaces of the wells by incubation with 0.5 ml of Eagle medium supplemented with 5 percent calf serum for 2 hours at 37°C. After completion of the incubation period, the medium was removed and the plates washed twice with 1 milliliter of distilled water per well leaving the plate covered with [$^{125}$I]fibrin.

A. aFGF Alone

2 microliter aliquots of 500 microliter detergent-solubilized cell extracts were added to 0.5 milliliter samples of 0.1 M Tris-HCl, pH 8.1 containing 125 micrograms of bovine serum albumin and 4 micrograms of plasminogen (prepared by lysine-Sepharose affinity chromatography of serum as described in Crowe et

* Commercially available growth medium, p. 38, "Gibco Laboratories Technical Brochure", Gibco Laboratories, Grand Island, New York; Tissue Culture Standards Comm., In Vitro, Vol. 6, No. 2, p. 93

** Commercially available Dulbecco's Phosphate Buffered Saline, p. 26, "Gibco Laboratories Technical Brochure"

*** Commercially available minimum essential medium - MEM alpha p. 56-57, "Gibco Laboratories Technical Brochure"

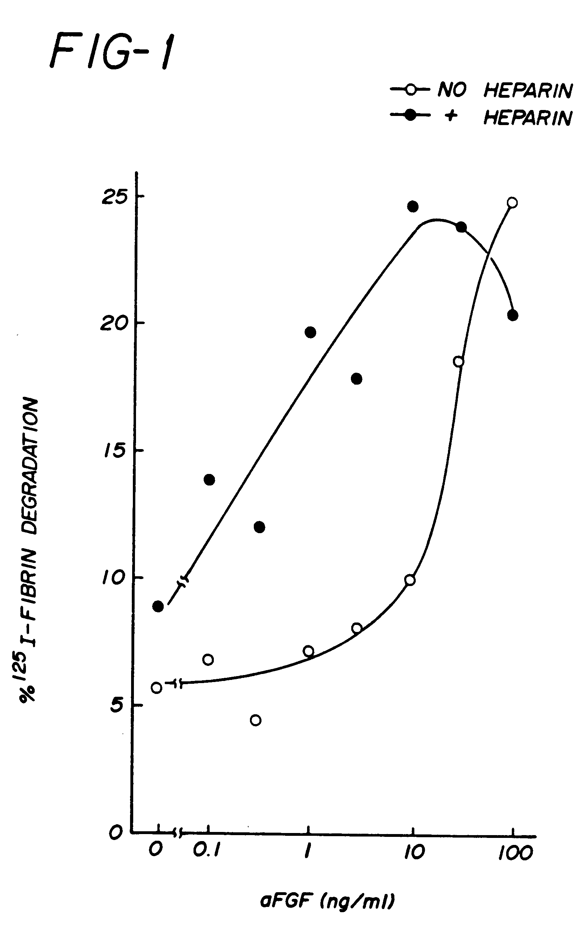

al., (1978) Experiments with Normal and Transformed Cells, Cold Spring Harbor Laboratory, Cold Spring Harbor, New York, p. 87) in dishes coated with [$^{125}$I]fibrin. After 1 hour incubation at 37°C, 100 microliter aliquots of fluid were taken and soluble plasmin-digested [$^{125}$I]fibrin fragments quantitated using a gamma spectrometer. Measurements on two replicate cultures were made and the average was taken as the value for each concentration of aFGF as well as for the controls. The radio-activity (cpm) released into assay media and its percentage of full digestion by trypsin (control) at different aFGF concentrations are listed in Table I. The plot of percent [$^{125}$I]fibrin degration is seen in Fig. 1.

## TABLE I

| aFGF | [$^{125}$I]Fibrin Degradation | | | |
|---|---|---|---|---|
| | Without Heparin | | With Heparin (50μg/ml) | |
| ng/ml | % | (cpm ) | % | (cpm) |
| 0 | 5.7 | (270) | 8.9 | (423) |
| 0.1 | 6.8 | (309) | 13.9 | (657) |
| 0.3 | 4.5 | (202) | 12.1 | (575) |
| 1 | 7.2 | (341) | 19.7 | (932) |
| 3 | 8.1 | (382) | 17.9 | (845) |
| 10 | 10.0 | (473) | 23.9 | (1133) |
| 30 | 18.0 | (879) | 24.7 | (1167) |
| 100 | 24.9 | (1176) | 20.5 | (967) |
| Trypsin control | 100.0 | (4741) | | |

B. aFGF plus Heparin

In a similar procedure, plasminogen activator activity was measured for varying concentrations of aFGF to which was added 50 micrograms of heparin (Porcine intestinal mucosa, Sigma, St. Louis, MO) per milliliter. The results are shown in Table I and in Fig. 1.

EXAMPLES II

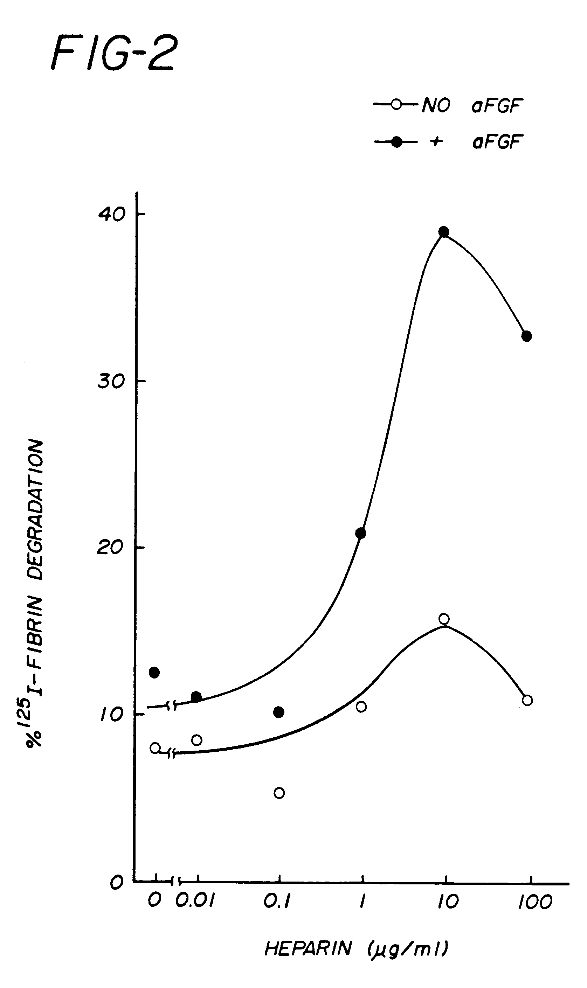

In a similar procedure, plasminogen activator activity was measured for varying concentrations of heparin alone and varying concentrations of heparin with 10 nanograms of aFGF per milliliter. The results are shown in Tables II and in Fig. 2.

6

## TABLE II

| Heparin | $[^{125}I]$ Fibrin Degradation | | | |
|---|---|---|---|---|
| | −aFGF | | +aFGF (10mg/ml) | |
| µg/ml | % | (cpm) | % | (cpm) |
| 0 | 8.0 | (673) | 12.5 | (1044) |
| 0.01 | 8.4 | (703) | 11.1 | (933) |
| 0.1 | 5.3 | (442) | 10.1 | (843) |
| 1 | 10.5 | (878) | 20.9 | (1746) |
| 10 | 15.8 | (1326) | 39.1 | (3271) |
| 100 | 10.9 | (913) | 32.8 | (2750) |
| Trypsin control | 100.0 | (8373) | | |

## Claims

1. The use of a composition comprising acidic fibroblast growth factor (aFGF) and heparin for the preparation of an intravenous medicament useful for inducing vascular repair.

2. A composition suitable for intravenous administration for inducing vascular repair comprising a therapeutic amount of acidic fibroblast growth factor (aFGF) and heparin, characterised in that the acidic fibroblast growth factor is in the range of 1µg to 100mg and heparin is in the range of 0.5 to 10mg in a milliliter of composition.

3. A composition constituted as in claim 2, for use in the treatment of thrombosis.

## Patentansprüche

1. Verwendung eines Präparates, umfassend einen sauren Fibroblasten-Wachstumsfaktor (aFGF) und Heparin zur Herstellung eines intravenösen Medikamentes, das nützlich ist, um eine vaskuläre Heilung auszulösen.

2. Präparat, das geeignet ist zur intravenösen Verabreichung, um eine vaskuläre Heilung auszulösen, umfassend eine therapeutische Menge eines sauren Fibroblasten-Wachstumsfaktors (aFGF) und Heparin, dadurch gekennzeichnet, daß der saure Fibroblasten-Wachstumsfaktor im Bereich von 1 mg bis 100 mg und Heparin im Bereich von 0,5 bis 10 mg in einem Milliliter Präparat liegen.

3. Masse nach Anspruch 2 zur Verwendung bei der Thrombosebehandlung.

## Revendications

1. Utilisation d'une composition comprenant un facteur de croissance acide des fibroblastes (FGFa) et de l'héparine pour la préparation d'un médicament intraveineux utile pour l'induction d'une réparation vasculaire.

2. Composition appropriée pour une administration intraveineuse pour l'induction d'une réparation vasculaire, comprenant une quantité thérapeutique d'un facteur de croissance acide des fibroblastes (FGFa) et de l'héparine, caractérisée en ce que la concentration du facteur de croissance acide des fibroblastes est de 1 μg à 100 mg et celle de l'héparine est de 0,5 à 10 mg dans un millilitre de composition.

3. Composition selon la revendication 2, pour une utilisation dans le traitement de la thrombose.

# FIG-1

# FIG-2

Legend:
- —○— NO aFGF
- —●— + aFGF

Y-axis: % $^{125}I$-FIBRIN DEGRADATION (0, 10, 20, 30, 40)

X-axis: HEPARIN (μg/ml) (0, 0.01, 0.1, 1, 10, 100)